# EUROPEAN PATENT SPECIFICATION

(11) **EP 0 792 110 B1**
(45) Date of publication and mention of the grant of the patent: **22.05.2002**
(21) Application number: 95943929.0
(22) Date of filing: 13.12.1995
(51) Int. Cl.: A23L 1/22, C07F 7/18, A23L 3/3454, A61K 7/16

(54) **SILICONE COMPOSITIONS**
SILIKON ENTHALTENDE MITTEL
COMPOSITIONS A BASE DE SILICONE

(30) Priority: 22.12.1994 GB 9425928
(43) Date of publication of application: 03.09.1997
(73) Proprietor: THE PROCTER & GAMBLE COMPANY, Cincinnati, Ohio 45202 (US)
(72) Inventor: HUGHES, Iain, Allan, Weybridge, Surrey KT13 8EQ (GB)
(74) Representative: Clemo, Nicholas Graham
(86) International application number: US9516675
(87) International publication number: WO9619119

(56) References cited:
- EP-A- 0 596 304
- EP-A- 0 612 517
- EP-A- 0 676 194
- EP-A- 0 717 978
- WO-A-94/01073
- WO-A-94/18940
- US-A- 3 723 491
- US-A- 4 136 250
- US-A- 4 157 384
- US-A- 4 725 575

## Description

The present invention relates to the use of dimethicone copolyols in lipophilic compositions, based on flavorants, perfumes, coolants or antimicrobial agents as lipophile, to improve residuality, impact and/or efficacy on surfaces treated therewith, for example teeth, dentures, skin, hair, laundry, dishware, working surfaces and the like.

### BACKGROUND

Lipophilic compositions such as flavor, perfume, coolant and disinfectant compositions are widely used either directly or in a variety of household products inclusive of cosmetics, oral and denture compositions, bleach, dishwashing, hard surface cleaning and laundry detergent products, etc. A common problem encountered with lipophilic compositions is that of improving surface substantivity or residuality of the lipophilic component. It would be desirable in many if not most household applications to enhance the surface residuality of the lipophile in order, for example, to provide increased flavor or perfume impact or increased antimicrobial efficacy.

Modern dental hygiene and denture preparations, for example, typically contain antiplaque and/or antitartar agents, as well as antimicrobial agents and flavorants. Antimicrobial action could affect plaque formation by either reducing the number of bacteria in the mouth/dentures or by killing those bacteria trapped in the film to prevent further growth and metabolism. Flavorants may alleviate the problem of bad breath via a deodorizing action. Some antimicrobial agents, e.g. menthol may, also serve as breath deodorizers. However, the efficacy of antimicrobial agents depends largely on their intraoral/denture retention, particularly their retention on the surface of the teeth or dentures where plaque is formed.

A typical disadvantage of known dental preparations is that only a relatively short time during which the teeth are being cleaned or the mouth is being rinsed is available for antimicrobial agents in the preparations to take effect. The problem is compounded by the fact that dentifrice preparations are used infrequently; most are used once or, perhaps, twice daily. Consequently, the long time period between brushings for a majority of the population provides optimum plaque forming conditions.

In many other personal and household applications, it would be desirable to provide enhanced surface substantivity. Laundry detergents, for example, would benefit by increasing perfume substantivity on fabrics so as to provide increased perfume impact on clothing after laundering or during use. Increased antimicrobial substantivity would also be beneficial from the viewpoint of reducing malodors associated with sweat or other soils. Enhanced perfume substantivity would also be valuable in fine fragrance and perfumed cosmetics. Enhanced coolant substantivity, on the other hand, would be beneficial in cough/cold products.

There has been a need, therefore, for developing lipophilic compositions which have improved surface residuality, impact and/or antimicrobial efficacy.

It is known to include silicones in dentifrice compositions, allegedly to coat the teeth and prevent cavities and staining. For instance, GB-A-689,679 discloses a mouthwash containing an organopolysiloxane for preventing adhesion of, or for removing tars, stains, tartar and food particles from the teeth. The mouthwash may include antiseptic compounds, such as thymol, and flavoring and perfuming agents.

US-A-2,806,814 discloses dental preparations including, in combination, a higher aliphatic acyl amide of an amino carboxylic acid compound as an active and a silicone compound. The patent notes that silicone compounds have been proposed for prevention of adhesion or to facilitate the removal of tars, stains, tartar and the like from teeth. The silicone compound is said to act as a synergist in improving the antibacterial and acid inhibiting activity of the active ingredient. Dimethyl polysiloxanes are said to be particularly effective. Flavoring oils and/or menthol may be included.

US-A-3624120 discloses quaternary ammonium salts of cyclic siloxane polymers for use as cationic surfactants, bactericides and as anticariogenic agents.

EP-A-612,517 and WO 94/01973 disclose emulsions which comprise a silicone phase and employ dimethicone copolyols as emulsifiers.

WO 94/18940 disclose sunscreen compositions, including examples which comprise cetyl dimethicone copolyol.

EP-A-717,978 and EP-A-676,194, which are relevant only under Art. 54(3) EPC, also disclose compositions comprising cetyl dimethicone copolyol.

EP-A-596,304 describes the use of other organopolysiloxanes for improving the spreadability of oils. One example includes cetyl dimethicone copolyol.

Accordingly, the present invention provides for improved surface-substantivity, impact and/or efficacy of flavor, perfume, coolant or antimicrobial agents in compositions containing them.

### SUMMARY OF THE INVENTION

The invention relates to the use of a dimethicone copolyol with a lipophile selected from flavorants, perfumes, physiological coolants, antimicrobial agents and mixtures thereof to provide improved surface residuality, wherein the dimethicone copolyol is selected from alkyl- and alkoxy-dimethicone copolyols having the formula (I). 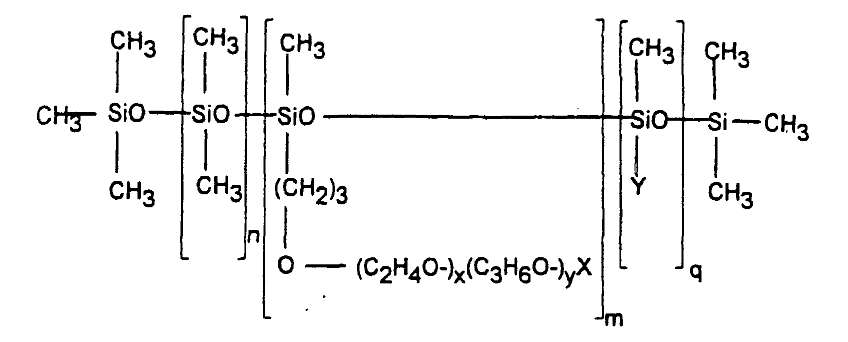 wherein X is selected from hydrogen, alkyl, alkoxy and acyl groups having from 1 to 16 carbon atoms, Y is selected from alkyl and alkoxy groups having from 8 to 22 carbon atoms, n is from 0 to 200, m is from 1 to 40, q is from 1 to 100, the molecular weight of the residue (C₂H₄O-)ₓ(C₃H₆O-)_{y}X is from 50 to 2000, preferably from 250 to 1000 and x and y are such that the weight ratio of oxyethylene:oxypropylene is from 100:0 to 0:100, preferably from 100:0 to 20:80.

All percentages and ratios herein are by weight of total composition, unless otherwise indicated.

In prefered embodiments, the dimethicone copolyol is selected from C₁₂ to C₂₀ alkyl dimethicone copolyols and mixtures thereof. Highly preferred is cetyl dimethicone copolyol marketed under the Trade Name Abil EM90. The dimethicone copolyol is generally present in a level of from 0.01% to 25%, preferably from 0.1 % to 5%, more preferably from 0.5% to 1.5% by weight.

In general terms, lipophiles (or lipophilic compounds) suitable for use herein are oil-like materials which are soluble or solubilisable in the dimethicone copolyol, preferably at a level of at least 1%, more preferably at least 5% by weight at 25°C. Preferred lipophilic compounds are selected from flavorants, perfumes, physiological cooling agents and antimicrobial compounds. The dimethicone copolyol acts to enhance the substantivity of the lipophilic compound to a surface treated therewith, thereby providing enhanced and/or sustained flavor, perfume or coolant impact and/or antimicrobial efficacy.

Lipophilic flavorants suitable for use herein comprise one or more flavor components selected from wintergreen oil, oregano oil, bay leaf oil, peppermint oil, spearmint oil, clove oil, sage oil, sassafras oil, lemon oil, orange oil, anise oil, benzaldehyde, bitter almond oil, camphor, cedar leaf oil, marjoram oil, citronella oil, lavendar oil, mustard oil, pine oil, pine needle oil, rosemary oil, thyme oil, cinnamon leaf oil, and mixtures thereof.

Lipophilic perfumes suitable for use herein comprise one or more known perfume components inclusive of natural products such as essential oils, absolutes, resins, etc., and synthetic perfume components such as hydrocarbons, alcohols, aldehydes, ketones, ethers, acids, esters, acetals, ketals, nitriles etc., including saturated and unsaturated compounds, aliphatic, carboxylic and heterocyclic compounds. Examples of perfume materials suitable for use herein include geranyl acetate, linalyl acetate, citronellyl acetate, dihydromyrcenyl acetate, terpinyl acetate, tricyclodecenyl acetate, tricyclodecenyl propionate, 2-phenylethyl acetate, benzyl acetate, benzyl salicylate, benzyl benzoate, styrallyl acetate, amyl salicylate, methyl dihydrojasmonate, phenoxyethyl isobutyrate, neryl acetate, trichloromethyl-phenylcarbinyl acetate, p-tertiary butyl-cyclohexyl acetate, isononyl acetate, cedryl acetate, vetiveryl acetate, benzyl alcohol, 2-phenylethanol, linalool, tetrahydrolinalool, citronellol, dimethylbenzylcarbinol, dihydromyrcenol, tetrahydromyrcenol, terpineol, eugenol, geraniol, vetiverol, 3-isocamphyl-cyclohexanol, 2-methyl-3-(p-tertiary butylphenyl)-propanol, 2-methyl-3-(p-isopropylphenyl)-propanol, 3-(p-tertiary butylphenyl)-propanol, nerol, alpha-n-amylcinnamic aldehyde, alpha-hexyl-cinnamic aldehyde, 4-(4-hydroxy-4-methylpentyl)-3-cyclohexenecarbaldehyde, 4-(4-methyl-3-pentenyl)-3-cyclohexenecarbaldehyde, 4-acetoxy-3-pentyltetrahydropyran, 2-n-heptyl-cyclopentanone, 3-methyl-2-pentylcyclopentanone, n-decanal, n-dodecanal, hydroxycitronellal, phenylacetaldehyde dimethyl acetal, phenylacetaldehyde diethyl acetal, geranonitrile, citronellonitrile, cedryl methyl ether, isolongifolanone, aubepine nitrile, aubepine, heliotropine, coumarin, vanillin, diphenyl oxide, ionones, methyl ionones, isomethyl ionones, irones, cis-3-hexenol and esters thereof, indane musks, tetralin musks, isochroman musks, macrocyclic ketones, macrolactone musks, ethylene brassylate, aromatic nitromusks and mixtures thereof.

Lipophilic antimicrobial compounds suitable for use herein include thymol, menthol, triclosan, 4-hexylresorcinol, phenol, eucalyptol, benzoic acid, benzoyl peroxide, butyl paraben, methyl paraben, propyl paraben, salicylamides, and mixtures thereof.

Physiological cooling agent suitable for use herein include carboxamides, menthane esters and menthane ethers, and mixtures thereof.

Suitable menthane ethers for use herein are selected from those with the formula: 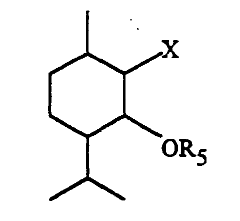 where R₅ is an optionally hydroxy substituted aliphatic radical containing up to 25 carbon atoms, preferably up to 5 carbon atoms, and where X is hydrogen or hydroxy, such as those commercially available under the trade name Takasago, from Takasago International Corporation. A particularly preferred cooling agent for use in the compositions of the present invention is Takasago 10 [3-1-menthoxy propan-1,2-diol (MPD)]. MPD is a monoglycerin derivative of 1-menthol and has excellent cooling activity.

The carboxamides found most useful are those described in US-A-4,136,163, January 23, 1979 to Wason et al., and US-A-4,230, 688, October 28, 1980 to Rawsell et al.

The level of lipophilic compound in compositions to which the invention is applicable is generally in the range from 0.01% to 10%, preferably from 0.05 % to 5%, more preferably from 0.1 % to 3 % by weight.

Compositions to which the invention applies optionally include one or more surfactants, these being especially preferred in lipophilic compositions for the purpose of solubilization of the lipophile and for providing improved efficacy. Suitable surfactants include non-soap anionic, nonionic, cationic, zwitterionic and amphoteric organic synthetic detergents. Many of these suitable agents are disclosed by Gieske et al. in US-A-4,051,234, September 27, 1977.

Examples of surfactants suitable for use herein include C₆-C₁₈ alkyl sulfates and alkyl ether sulfates ethoxylated with from 0.5 to 20 moles of ethylene oxide per mole; anionic sulfonates inclusive of C₅-C₂₀ linear alkylbenzene sulfonates, alkyl ester sulfonates, C₆-C₂₂ primary or secondary alkane sulfonates, C₆-C₂₄ olefin sulfonates, sulfonated polycarboxylic acids, alkyl glycerol sulfonates, fatty acyl glycerol sulfonates, and mixtures thereof; anionic carboxylates inclusive of primary and secondary C₆ to C₁₈ alkyl carboxylate, ethoxy carboxylate and polyethoxy polycarboxylate surfactants having an average degree of ethoxylation of from about 0 to about 10; C₅-C₁₇ sarcosinates such as sodium cocoylsarcosinate, sodium lauroyl sarcosinate (Hamposyl-95 ex W. R. Grace); condensation products of ethylene or propylene oxide with fatty acids, fatty alcohols, fatty amides, polyhydric alcohols (e.g. sorbitan monostearate, sorbitan oleate), alkyl phenols (e.g. Tergitol) and polypropyleneoxide or polyoxybutylene (e.g. Pluronics); alkylpolysaccharides as disclosed in US-A-4,565,647; amine oxides such as dimethyl cocamine oxide, dimethyl lauryl amine oxide and cocoalkyldimethyl amine oxide (Aromox); polysorbates such as Tween 40 and Tween 80 (Hercules); sorbitan stearates, sorbitan monooleate, etc; cationic surfactants such as cetyl pyridinium chloride, cetyl trimethyl ammonium bromide, diisobutyl phenoxy ethoxy ethyl-dimethyl benzyl ammonium chloride and coconut alkyl trimethyl ammonium nitrate.

Highly preferred herein from the view point of lipophile solubilization are the nonionic surfactants. One class of nonionic surfactant suitable for use herein are those having the general formula: 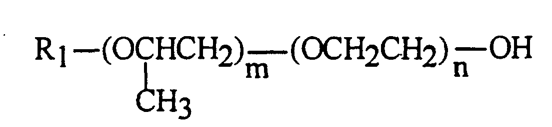 in which R₁ is an alk(en)yl or alk(en)yl phenyl group having 8 to 22, preferably 10 to 20 carbon atoms ion the alk(en)yl moiety and m and n represent weight-averages in the range 0-80 and 2-80 respectively. Shorter chain length alkyl groups are generally to be avoided for efficacy reasons and because unreacted fatty alcohol in such surfactants is a source of malodour and occasionally of skin irritation. It will be understood that surfactants of this type are usually mixtures of varying degrees of ethoxylation / propoxylation, accordingly m and n represent the respective weight-averages of the number of propoxylate and ethoxylate groups. Nonionic surfactants of the above general type include mixed alkoxylates in which m and n are both in the range from about 2 to about 80, with m preferably being in the range from about 2 to about 20, more preferably from about 3 to about 10 and with n preferably being in the range from about 2 to about 60, more preferably from about 5 to about 50. One such material is PPG-5-ceteth-20 (available from Croda Inc as Procetyl AWS), where m and n have the values 5 and 20 respectively. Other suitable nonionic surfactants include polyethoxylated surfactants, e.g. ethoxylated alkylphenol ethers, particularly octyl- and nonylphenol ethers containing 8-16 EO; ethoxylated aliphatic C₈-C₂₀ alcohols, which may be linear or branched and contain 8-16, preferably 9-15 EO; and ethoxylated hydrogenated castor oils.

In general, the ratio of surfactant to the perfume, coolant or other oily material will be in the range of from 50:1 to 1:10, preferably from 20:1 to 1:2, more preferably from 10:1 to 1:1.

The following Examples further describe and demonstrate the preferred embodiments within the scope of the present invention.

### EXAMPLES I TO IV

The following are representative perfume, flavour, coolant and antimicrobial compositions to which the invention applies. The percentages are by weight of total composition.

| | I | II | III | IV |
|---|---|---|---|---|
| PPG-5-ceteth-20 | 3.0 | 3.0 | 4.5 | 3.0 |
| PEG-40 hydrogenated castor | - | 1.8 | 4.5 | 3.0 |
| oil | | | | |
| Trideceth-12 | 2.0 | - | - | - |
| Trideceth-9 | - | 2.0 | - | 3.0 |
| Flavor⁵ | 2.0 | | | 3.0 |
| Parfume⁶ | - | 3.0 | - | - |
| Trimethyl butanamide | 0.3 | 0.5 | - | - |
| Triclosan | - | - | 1.0 | 0.5 |
| Abil EM90⁴ | 1.0 | 1.5 | 5.0 | 1.0 |
| Water | <------ ------- to 100% ---- -------> | | | |

| | | | | |
|---|---|---|---|---|
| 4. Cetyl dimethicone copolyol | | | | |
| 5. Peppermint based flavour. | | | | |
| 6. Perfume is a complex mixture of ingredients used primarily for olfactory purposes. | | | | |

The perfume flavor, coolant and/or antimicrobial compositions of Example I to IV display improved surface-substantivity, impact and/or efficacy.

## Claims

1. Use of a dimethicone copolyol with a lipophile selected from flavorants, perfumes, physiological coolants and antimicrobial agents to provide improved surface residuality, wherein the dimethicone copolyol is selected from alkyl- and alkoxy-dimethicone copolyols having the formula (I): 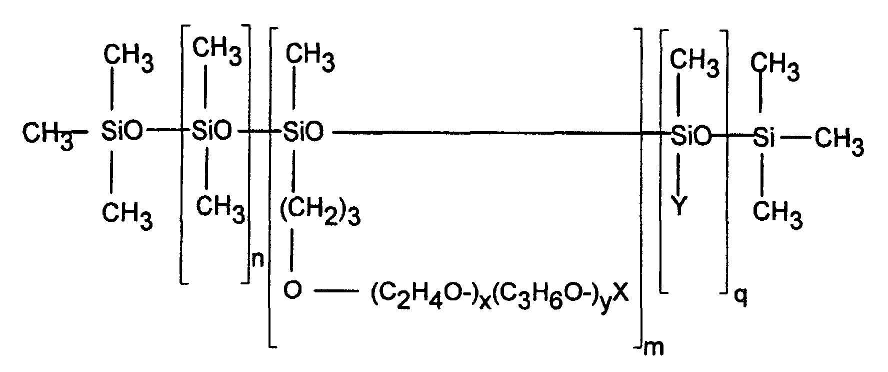 wherein X is selected from hydrogen, alkyl, alkoxy and acyl groups having from 1 to 16 carbon atoms, Y is selected from alkyl and alkoxy groups having from 8 to 22 carbon atoms, n is from 0 to 200, m is from 1 to 40, q is from 1 to 100, the molecular weight of the residue (C₂H₄O-)ₓ(C₃H₆O-)_{y}X is from 50 to 2000, and x and y are such that the weight ratio of oxyethylene:oxypropylene is from 100:0 to 0:100.

2. Use according to Claim 1 wherein the dimethicone copolyol is selected from C₁₂ to C₂₀ alkyl dimethicone copolyols and mixtures thereof.

3. Use according to Claim 1 or 2 wherein the dimethicone copolyol is cetyl dimethicone copolyol.

4. Use according to any of Claims 1 to 3 wherein the dimethicone copolyol and the lipophile are provided in a composition comprising from 0.01% to 25%, preferably from 0.1% to 5% by weight of the dimethicone copolyol.

5. Use according to Claim 4 wherein the composition further comprises one or more surfactants.

6. Use according to Claim 5 wherein the composition comprises a nonionic surfactant.

7. Use according to any of Claims 1 to 6 wherein the lipophile is a flavour component selected from wintergreen oil, oregano oil, bay leaf oil, peppermint oil, spearmint oil, clove oil, sage oil, sassafras oil, lemon oil, orange oil, anise oil, benzaldehyde, bitter almond oil, camphor, cedar leaf oil, marjoram oil, citronella oil, lavender oil, mustard oil, pine oil, pine needle oil, rosemary oil, thyme oil, cinnamon leaf oil, and mixtures thereof.

8. Use according to any of Claims 1 to 6 wherein the lipophile is a perfume component selected from geranyl acetate, linalyl acetate, citronellyl acetate, dihydromyrcenyl acetate, terpinyl acetate, tricyclodecenyl acetate, tricyclodecenyl propionate, 2-phenylethyl acetate, benzyl acetate, benzyl salicylate, benzyl benzoate, styrallyl acetate, amyl salicylate, methyl dihydrojasmonate, phenoxyethyl isobutyrate, neryl acetate, trichloromethyl-phenylcarbinyl acetate, p-tert-butyl-cyclohexyl acetate, isononyl acetate, cedryl acetate, vetiveryl acetate, benzyl alcohol, 2-phenylethanol, linalool, tetrahydrolinalool, citronellol, dimethylbenzylcarbinol, dihydromyrcenol, tetrahydromyrcenol, terpineol, eugenol, geraniol, vetiverol, 3-isocamphyl-cyclohexanol, 2-methyl-3-(p-tert-butylphenyl)-propanol, 2-methyl-3-(p-isopropylphenyl)-propanol, 3-(p-tert-butylphenyl)-propanol, nerol, alpha-n-amylcinnamic aldehyde, alpha-hexyl-cinnamic aldehyde, 4-(4-hydroxy-4-methylpentyl)-3-cyclohexenecarbaldehyde, 4-(4-methyl-3-pentenyl)-3-cyclohexenecarbaldehyde, 4-acetoxy-3-pentyltetrahydropyran, 2-n-heptyl-cyclopentanone, 3-methyl-2-pentyl-cyclopentanone, n-decanal, n-dodecanal, hydroxycitronellal, phenylacetaldehyde dimethyl acetal, phenylacetaldehyde diethyl acetal, geranonitrile, citronellonitrile, cedryl methyl ether, isolongifolanone, aubepine nitrile, aubepine, heliotropine, coumarin, vanillin, diphenyl oxide, ionones, methyl ionones, isomethyl ionones, irones, cis-3-hexenol and esters thereof, indane musks, tetralin musks, isochroman musks, macrocyclic ketones, macrolactone musks, ethylene brassylate, aromatic nitromusks and mixtures thereof.

## Patentansprüche

1. Verwendung eines Dimethiconcopolyols mit einem Lipophil, welches unter Geschmacksmitteln, Parfums, physiologischen Kühlungsmitteln und antimikrobiellen Mitteln ausgewählt ist, um das Verbleiben auf der Oberfläche zu verbessern, wobei der Dimethiconcopolyol unter Alkyl- und Alkoxydimethiconcopolyolen der Formel (I): 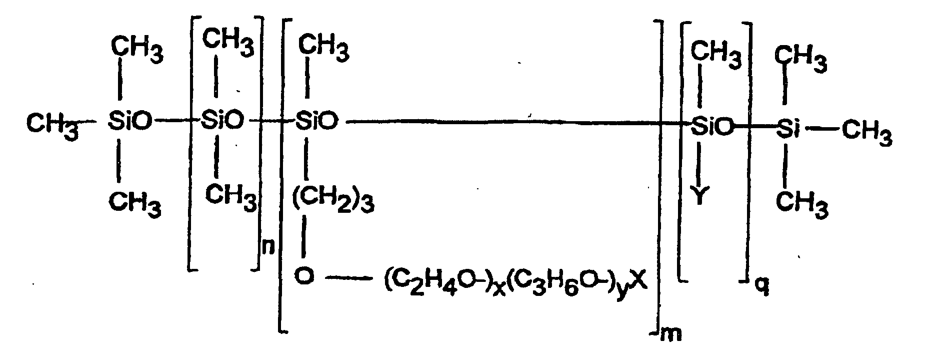 ausgewählt ist, worin X unter Wasserstoff, Alkyl-, Alkoxy- und Acylgruppen mit 1 bis 16 Kohlenstoffatomen ausgewählt ist, Y unter Alkyl- und Alkoxygruppen mit 8 bis 22 Kohlenstoffatomen ausgewählt ist, n von 0 bis 200 beträgt, m von 1 bis 40 beträgt, q von 1 bis 100 beträgt, das Molekulargewicht des Restes (C₂H₄O-)ₓ(C₃H₆O-)_{y}X von 50 bis 2.000 beträgt und x und y derart sind, daß das Gewichtsverhältnis von Oxyethylen:Oxypropylen von 100:0 bis 0:100 beträgt.

2. Verwendung nach Anspruch 1, wobei der Dimethiconcopolyol unter C₁₂-C₂₀Alkyldimethiconcopolyolen und Gemischen hievon ausgewählt ist.

3. Verwendung nach Anspruch 1 oder 2, wobei der Dimethiconcopolyol Cetyldimethiconcopolyol ist.

4. Verwendung nach einem der Ansprüche 1 bis 3, wobei der Dimethiconcopolyol und das Lipophil in einer Zusammensetzung bereitgestellt werden, welche 0,01 Gew.-% bis 25 Gew.-%, vorzugsweise 0,1 Gew.-% bis 5 Gew.-% des Dimethiconcopolyols umfaßt.

5. Verwendung nach Anspruch 4, wobei die Zusammensetzung ferner ein oder mehrere grenzflächenaktive Mittel umfaßt.

6. Verwendung nach Anspruch 5, wobei die Zusammensetzung ein nichtionisches grenzflächenaktives Mittel umfaßt.

7. Verwendung nach einem der Ansprüche 1 bis 6, wobei das Lipophil eine Geschmackstoffkomponente ist, welche unter Wintergrünöl, Oreganumöl, Lorbeerblattöl, Pfefferminzöl, Spearminzenöl, Nelkenöl, Salbeiöl, Sassafrasöl, Zitronenöl, Orangenöl, Anisöl, Benzaldehyd, Bittermandelöl, Kampfer, Zederblattöl, Majoranöl, Zitronellaöl, Lavendelöl, Senföl, Pinienöl, Piniennadelöl, Rosmarinöl, Thymianöl, Zimtblattöl und Gemischen hievon ausgewählt ist.

8. Verwendung nach einem der Ansprüche 1 bis 6, wobei das Lipophil eine Parfümkomponente ist, welche unter Geranylacetat, Linalylacetat, Citronellylacetat, Dihydromyrcenylacetat, Terpinylacetat, Tricyclodecenylacetat, Tricyclodecenylpropionat, 2-Phenylethylacetat, Benzylacetat, Benzylsalicylat, Benzylbenzoat, Styrallylacetat, Amylsalicylat, Methyldihydrojasmonat, Phenoxyethylisobutyrat, Nerylacetat, Trichlormethyl-phenylcarbinylacetat, p-tert.-Butylcyclohexylacetat, Isononylacetat, Cedrylacetat, Vetiverylacetat, Benzylalkohol, 2-Phenylethanol, Linalool, Tetrahydrolinalool, Citronellol, Dimethylbenzylcarbinol, Dihydromyrcenol, Tetrahydromyrcenol, Terpineol, Eugenol, Geraniol, Vetiverol, 3-Isocamphyl-cyclohexanol, 2-Methyl-3-(p-tert.-butylphenyl)-propanol, 2-Methyl-3-(p-isopropylphenyl)-propanol, 3-(p-tert.-Butylphenyl)-propanol, Nerol, alpha-n-Amylzimtsäurealdehyd, alpha-Hexylzimtsäurealdehyd, 4-(4-Hydroxy-4-methylpentyl)-3-cyclohexencarbaldehyd, 4-(4-Methyl-3-pentenyl)-3-cyclohexencarbaldehyd, 4-Acetoxy-3-pentyltetrahydropyran, 2-n-Heptyl-cyclopentanon, 3-Methyl-2-pentyl-cyclopentanon, n-Decanal, n-Dodecanal, Hydroxycitronellal, Phenylacetaldehyddimethylacetal, Phenylacetaldehyddiethylacetal, Geranonitril, Citroneilonitril, Cedrylmethylether, Isolongifolanon, Aubepinnitril, Aubepin, Heliotropin, Coumarin, Vanillin, Diphenyloxid, Iononen, Methyliononen, Isomethyliononen, Ironen, cis-3-Hexenol und dessen Estern, Indanmoschus, Tetralinmoschus, Isochromanmoschus, makrocyclischen Ketonen, Makrolactonmoschus, Ethylenbrassylat, aromatischem Nitromoschus und Gemischen hievon ausgewählt ist.

## Revendications

1. Utilisation d'un diméthicone-copolyol avec un agent lipophile choisi parmi des agents aromatisants, des parfums, des agents réfrigérants physiologiques et des agents antimicrobiens, pour assurer une rémanence de surface améliorée, où le diméthicone-copolyol est choisi parmi des alkyl- et alcoxy-diméthicone-copolyols répondant à la formule (I) : 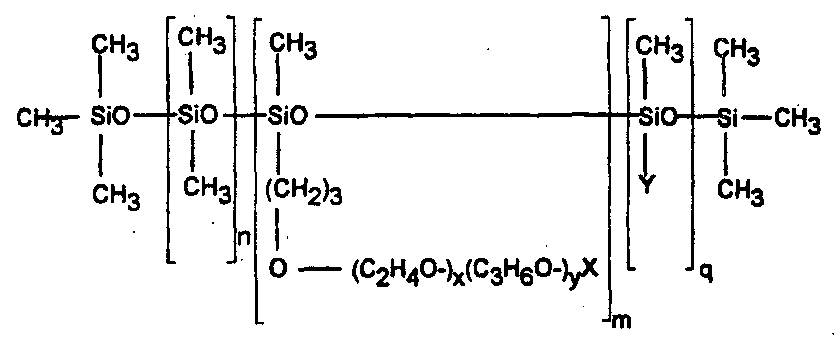 dans laquelle X est choisi parmi l'hydrogène, des groupes alkyle, alcoxy et acyle ayant 1 à 16 atomes de carbone, Y est choisi parmi des groupes alkyle et alcoxy ayant 8 à 22 atomes de carbone, n vaut entre 0 et 200, m vaut entre 1 et 40, q vaut entre 1 et 100, le poids moléculaire du résidu (C₂H₄O-)ₓ(C₃H₆O-)_{y}X est compris entre 50 et 2000, et x et y sont tels que le rapport pondéral oxyéthylène:oxypropylène est de 100:0 à 0:100.

2. Utilisation selon la revendication 1, dans laquelle le diméthicone-copolyol est choisi parmi des alkyl-diméthicone-copolyols en C₁₂ à C₂₀ et leurs mélanges.

3. Utilisation selon la revendication 1 ou 2, dans laquelle le diméthicone-copolyol est un cétyl-diméthicone-copolyol.

4. Utilisation selon l'une quelconque des revendications 1 à 3, dans laquelle le diméthicone-copolyol et l'agent lipophile sont présents dans une composition comprenant 0,01% à 25%, de préférence 0,1% à 5%, en poids du diméthicone-copolyol.

5. Utilisation selon la revendication 4, dans laquelle la composition comprend en outre un ou plusieurs agents tensioactifs.

6. Utilisation selon la revendication 5, dans laquelle la composition comprend un agent tensioactif non ionique.

7. Utilisation selon l'une quelconque des revendications 1 à 6, dans laquelle l'agent lipophile est un composant aromatisant choisi parmi l'essence de wintergreen, l'essence d'origan, l'essence de feuille de laurier, l'essence de menthe poivrée, l'essence de menthe verte, l'essence de girofle, l'essence de sauge, l'essence de sassafras, l'essence de citron, l'essence d'orange, l'essence d'anis, le benzaldéhyde, l'essence d'amande amère, le camphre, l'essence d'aiguilles de cèdre, l'essence de marjolaine, l'essence de citronnelle, l'essence de lavande, l'essence de moutarde, l'essence de pin, l'essence d'aiguilles de pin, l'essence de romarin, l'essence de thym, l'essence de cannelle, et leurs mélanges.

8. Utilisation selon l'une quelconque des revendications 1 à 6, dans laquelle l'agent lipophile est un composant de parfum choisi parmi l'acétate de géranyle, l'acétate de linalyle, l'acétate de citronellyle, l'acétate de dihydromyrcényle, l'acétate de terpinyle, l'acétate de tricyclodécényle, le propionate de tricyclodécényle, l'acétate de 2-phényléthyle, l'acétate de benzyle, le salicylate de benzyle, le benzoate de benzyle, l'acétate de styrallyle, le salicylate d'amyle, le dihydrojasmonate de méthyle, l'isobutyrate de phénoxyéthyle, l'acétate de néryle, l'acétate de trichlorométhyl-phénylcarbinyle, l'acétate de p-tert-butyl-cyclohexyle, l'acétate d'isononyle, l'acétate de cédryle, l'acétate de vétivéryle, l'alcool benzylique, le 2-phényléthanol, le linalol, le tétrahydrolinalol, le citronellol, le diméthylbenzylcarbinol, le dihydromyrcénol, le tétrahydromyrcénol, le terpinéol, l'eugénol, le géraniol, le vétivérol, le 3-isocamphylcyclohexanol, le 2-méthyl-3-(p-tert-butylphényl)-propanol, le 2-méthyl-3-(p-isopropylphényl)propanol, le 3-(p-tert-butylphényl)-propanol, le nérol, l'aldéhyde alpha-n-amylcinnamique, l'aldéhyde alpha-hexyl-cinnamique, le 4-(4-hydroxy-4-méthylpentyl)-3-cyclohexènecarbaldéhyde, le 4-(4-méthyl-3-pentényl)-3-cyclohexène-carbaldéhyde, le 4-acétoxy-3-pentyltétrahydropyranne, la 2-n-heptyl-cyclopentanone, la 3-méthyl-2-pentyl-cyclopentanone, le n-décanal, le n-dodécanal, l'hydroxycitronellal, le phénylacétaldéhyde-diméthylacétal, le phénylacétaldéhyde-diéthylacétal, le géranonitrile, le citronellonitrile, l'éther méthylique de cédryle, l'isolongifolanone, l'aubépine-nitrile, l'aubépine, l'héliotropine, la coumarine, la vanilline, l'oxyde de diphényle, des ionones, des méthyl-ionones, des isométhyl-ionones, des irones, le cis-3-hexénol et des leurs esters, des muscs d'indane, des muscs de tétraline, des muscs d'isochromane, des cétones macrocycliques, des muscs de macrolactone, le brassylate d'éthylène, des nitromuscs aromatiques, et leurs mélanges.
